# EUROPÄISCHE PATENTSCHRIFT

(11) **EP 2 338 414 B1**
(45) Veröffentlichungstag und Bekanntmachung des Hinweises auf die Patenterteilung: **28.11.2012**
(21) Anmeldenummer: 10192885.1
(22) Anmeldetag: 29.11.2010
(51) Int. Cl.: A61B 5/103, A61N 1/37, G01R 33/28

(54) **Lagesensor zur MRT-Erkennung**
Position sensor for MRT detection
Capteur de position pour la reconnaissance MRT

(30) Priorität: 22.12.2009 US 288864 P
(43) Veröffentlichungstag der Anmeldung: 29.06.2011
(73) Patentinhaber: Biotronik CRM Patent AG, 6341 Baar (CH)
(72) Erfinder: Dörr, Thomas, 12437, Berlin (DE); Weiss, Ingo, 12435, Berlin (DE)
(74) Vertreter: Lindner-Vogt, Karin L.

(56) Entgegenhaltungen:
- EP-A1- 1 731 088
- WO-A1-2008/026970
- US-A1- 2005 080 461
- US-A1- 2008 154 342

## Beschreibung

Die Erfindung bezieht sich auf eine Vorrichtung zum Erkennen von elektromagnetischen Feldern, wie sie bei Untersuchungen mit bildgebenden Kemspintomographie- (im folgenden MRT- oder MRI-) Geräten auftreten.

Obwohl MRI-Untersuchungen einen immer höheren Stellenwert in der diagnostischen Medizin erhalten, ist ein Teil der Patienten für MRI-Untersuchungen kontraindiziert. Eine solche Kontraindikation kann durch ein mindestens teilweise implantiertes medizinisches Gerät (im Folgenden auch Implantat oder IMD) gegeben sein.

Um MRI-Untersuchungen dennoch zu ermöglichen, sind verschiedene Ansätze, die sich entweder auf die Durchführung der MRI-Untersuchung oder auf das implantierbare medizinische Gerät beziehen, bekannt.

Der bisherige Stand der Technik stellt zwar Methoden zur Erkennung von MRI-Feldern bereit, diese beruhen aber häufig auf einzelnen Messmethoden.

So beschreibt die US 2008/0154342 ein Verfahren unter Ausnutzung eines GMR-Sensors (Giant Magnetic Resistance), um problematische Magnetfelder von MRT-Geräten zu erkennen. Damit verlässt sich diese Methode auf einen einzigen Parameter, die gemessene Feldstärke.

Die WO2008/026970A1 beschreibt Vorrichtungen und ein Verfahren zum Bestimmen von Variationen von medizinischen Parametern in Abhängigkeit von der Zeit.

Aufgabe der Erfindung ist es, eine Vorrichtung für medizinische Geräte und implantierbare medizinische Geräte bereitzustellen, die die Nachteile des Stands der Technik beheben und eine Verifizierung der Erkennung von MRI-Feldern erlauben.

Die Aufgabe wird durch ein implantierbares medizinisches Gerät (IMD) mit den Merkmalen des Anspruchs 1 gelöst.

Dabei beinhaltet das IMD mindestens: eine Einheit zur Detektion von MRI-Störfeldern, wobei die Einheit über mindestens einen Sensor und/oder Indikator für MRI-typische elektromagnetische Störfelder verfügt, eine Steuereinheit die mit der Einheit zur Detektion von elektromagnetischen Störfeldern verbindbar ist, mindestens beinhaltend eine Diagnoseeinheit und/oder mindestens beinhaltend eine Therapieeinheit, wobei die Einheit zur Detektion von MRI-Störfeldern zusätzlich einen Lagesensor aufweist und die Einheit zur Detektion von MRI-Störfeldern nur dann eine Störung durch MRI-Störfelder erkennt, wenn neben zumindest einem Sensor und/oder zumindest einem Indikator für MRI-typische elektromagnetische Störfelder der Lagesensor eine liegende Positur des Patienten anzeigt. Unter liegender Positur ist eine horizontale Lage des Patienten zu verstehen. Unter Indikatoren für MRI-Störfelder sind Messungen zu verstehen, die auf elektromagnetische Störfelder schließen lassen.

Mit MRI-Störfeldern sind solche elektromagnetischen Felder gemeint, die in der Umgebung eines MRI-Gerätes durch das MRI-Gerät hervorgerufen werden. MRI-typische Störfelder sind zum Beispiel, aber nicht beschränkt auf, ein statisches Magnetfeld, ein Gradientenfeld und ein RF-Feld (elektromagnetisches Radiofrequenzfeld). Die Diagnoseeinheit kann beispielsweise, aber nicht beschränkt auf, eine Einheit zur Bestimmung von physikalischen und/oder chemischen und/oder biologischen Parameter sein und die Einheit zur Therapie kann beispielsweise, aber nicht beschränkt auf eine Einheit zur Abgabe von elektrischen Impulsen und/oder chemischen Substanzen und/oder mechanischen Kräften.

Ist die Spezifität der MRT-Erkennung jedoch eingeschränkt, so besteht das erhebliche Risiko, dass das Implantat auch außerhalb einer MRT-Umgebung durch andere Umgebungseinflüsse in einen MRT-Mode geschaltet wird und dadurch ebenfalls eine Patientengefährdung entsteht.

In einer liegenden Position (im Schlaf) können viele der mit einem MRT verwechselbaren Umgebungsbedingungen jedoch ausgeschlossen werden, so dass die zusätzliche Auswertung der Lageinformation für die MRT-Erkennung eine deutliche Spezifitätssteigerung bewirkt.

Es ist bevorzugt, dass der Lagesensor sich selber kalibriert, wobei eine liegende Positur entweder automatisch erkannt wird und/oder die Kalibrierung über ein externes Gerät, wie, aber nicht beschränkt auf, ein Patientengerät und/oder Programmiergerät, ausgelöst wird und/oder die liegende Positur über ein externes Gerät, wie, aber nicht beschränkt auf, ein Patientengerät und/oder Programmiergerät, bestätigt wird.

Besonders bevorzugt wird, dass die Kalibrierung des Lagesensors nach Auswertung der Uhrzeit erfolgt, vorzugsweise eine Kalibrierung während des Nachtschlafes.

Des Weiteren wird bevorzugt, dass zur Identifikation einer liegenden Positur die täglichen Phasen mit minimaler Herzfrequenz ausgewertet werden. So kann beispielsweise der Nachtschlaf erkannt werden, der bevorzugt zu einer Kalibrierung verwendet wird.

Bevorzugt wird auch, dass der Nachtschlaf durch die Auswertung der Herzfrequenzvariabilität und deren zirkadianer Rhythmik erkannt wird.

Ebenfalls bevorzugt wird, dass der Nachtschlaf, durch eine Auswertung der Atemfrequenz erkannt wird.

Auch wird bevorzugt, dass der Nachtschlaf durch eine Auswertung der Patientenaktivität erkannt wird.

Bevorzugt ist auch, dass der Nachtschlaf durch eine Auswertung mindestens eines EEG-Parameters erkannt wird. Ein EEG ist auch als Elektroenzephalographie bekannt.

Des Weiteren wird bevorzugt, dass eine Erkennung von MRI-Störfeldern nur innerhalb eines vorbestimmbaren Zeitfensters eine vorbestimmbare Reaktion des IMD initiiert.

Auch wird bevorzugt, dass die Einheit zur Detektion von MRI-Störfeldern mindestens einen der folgenden Sensoren und/oder Indikatoren umfasst: einen GMR-Sensor, einen MagFET-Sensor, einen Hallsensor, einen elektrooptische Wandler als Indikator, die Überwachung von Batteriespannungen während Kondensatorladeprozessen als Indikator, die Detektion von RF-Feldem als Indikator, die Detektion von magnetischen Grandientenfeldem als Indikator, und die Detektion von durch elektromagnetische Felder induzierten Strömen als Indikator.

Ebenfalls bevorzugt wird, dass zumindest eine der folgenden Maßnahmen bei der Erkennung von MRI-Störfeldern durch die Einheit zur Detektion von MRI-Störfeldern initiiert wird: das Wechseln in einen MRI-sicheren Zustand, ein verlängertes Verbleiben in einem MRI-sicheren oder gegenüber elektromagnetischen Störfeldern unempfindlichen Zustand, die Abgabe von elektromagnetischen Pulsen zur Signalisierung, dass ein IMD im elektromagnetischen Feld vorhanden ist, zur Signalisierung an ein MRI-Gerät, mit der Möglichkeit, neben der Störung auch Informationen auf diese Weise zu übertragen und auf dem Bildschirm des MRI-Gerät sichtbar zu machen, und die Abgabe einer Therapie und/oder Detektion von elektrischen Zuständen des Gewebes nur in Zeitfenstem erlaubt ist, in denen keine elektromagnetischen Störfelder erkannt werden und/oder eine Rekonstruktion einer Messung für die Bereiche durchgeführt wird, in denen die Detektion aufgrund von erkannten elektromagnetischen Störfeldern nicht erlaubt ist.

Des Weiteren wird die Aufgabe durch eine Vorrichtung zur Erhöhung der Spezifität bei der Erkennung von MRI-Störfeldern gelöst, wobei für eine Erkennung eines MRI-Störfeldes neben der Erkennung durch mindestens einen Sensor und/oder mindestens einen Indikator für MRI-typische elektromagnetische Störfelder auch die liegende Positur des Implantatträgers erkannt wird.

Das Verfahren kann beispielsweise mit einem IMD, wie oben beschrieben, ausgeführt werden Einige Aspekte der Erfindung werden in den Figuren 1 -3 dargestellt.
- Fig. 1: Schematische Darstellung des Ablaufs einer MRI-Untersuchung
- Fig. 2: Vereinfachtes Blockschaltbild eines erfindungsgemäßen IMD.
- Fig. 3: Variante eines vereinfachten Blockschaltbilds eines erfindungsgemäßen elektronischen Implantats.

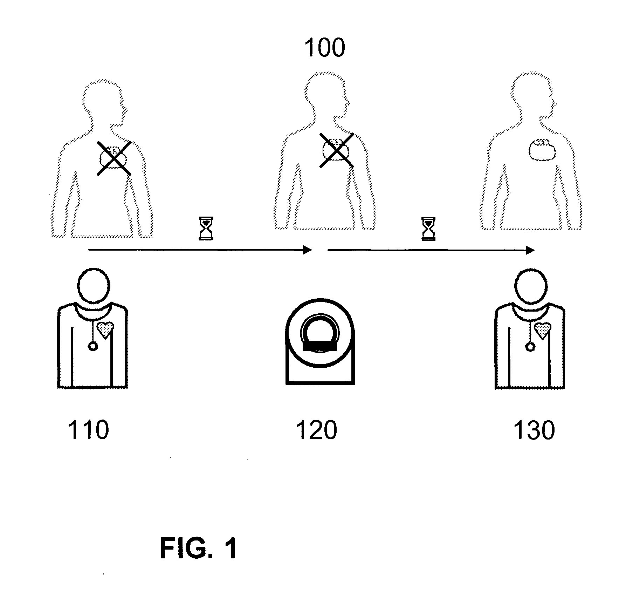

In Figur 1 ist die Ausgangssituation am Beispiel eines ICD (implantierbarer Defibrillator/Kardioverters) dargestellt. Der ICD-Patient (100) wird vor der geplanten MRT-Untersuchung von einem Kardiologen nachgesorgt und der ICD ausgeschaltet (110). Mit einer zeitlichen Verzögerung von Stunden bis Tagen erfolgt die MRT-Untersuchung bei einem Radiologen (120). Nach einer weiteren Verzögerung wird der Patient wieder vom Kardiologen (130) betreut und der ICD wieder eingeschaltet. Während der gesamten Zeit von (110) bis (130) ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung, in Kauf genommen.

Ebenso ist der finanzielle und logistische Aufwand einer solchen Prozedur sehr hoch und schließt in vielen Fällen die Notfallanwendung eines MRT aus.

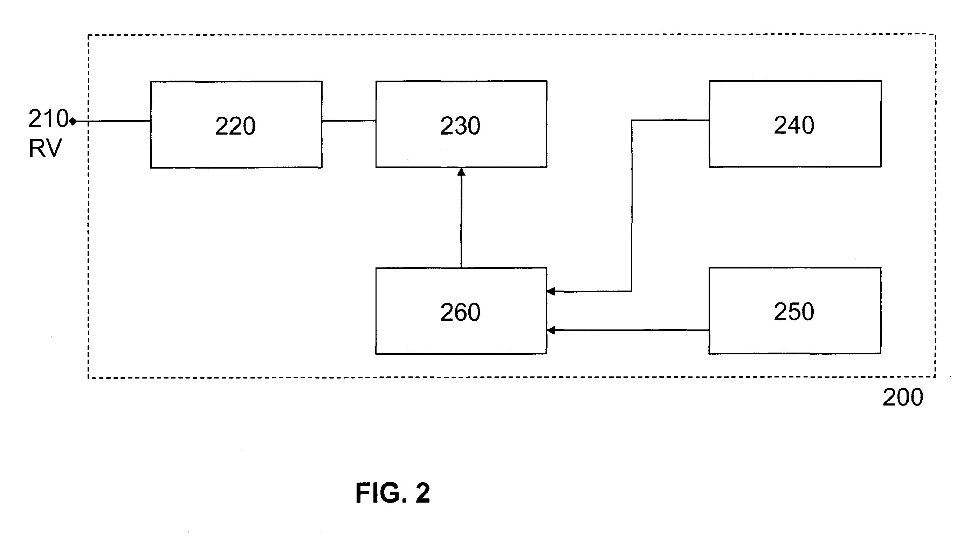

Die Figur 2 zeigt ein vereinfachtes Blockschaltbild eines elektronischen Implantates (200; z. B. Einkammer-Herzschrittmacher). Dieses besitzt eine Wahrnehmungs- und Stimulationseinheit (220), verbunden mit dem rechtsventrikulären Elektrodenanschluss (210) und einer Zeitgebereinheit (230) zur bedarfsgesteuerten Stimulation des Herzens.

Zusätzlich beinhaltet das elektronische Implantat mindestens eine Einheit zur Erkennung einer MRT-Umgebung (240), wie zum Beispiel, aber nicht beschränkt auf, einen GMR-Sensor (240; giant magnetoresistance).

Erfindungsgemäß wird dieses Blockschaltbild um einen dreidimensionalen Lagesensor (250) erweitert. Die beiden Sensorsignale werden in einer speziellen Steuereinheit ausgewertet und immer dann, wenn der MRT-Sensor (240) eine MRT-Umgebung anzeigt und der Lagesensor (250) eine liegende Position des Patienten signalisiert, wird mittels der Steuereinheit (260) die Zeitgebereinheit (230) in eine vorprogrammierte MRT-sichere Betriebsart, wie zum Beispiel, aber nicht beschränkt auf, V00 oder D00 bei schrittmacherabhängigen Patienten umgeschaltet.

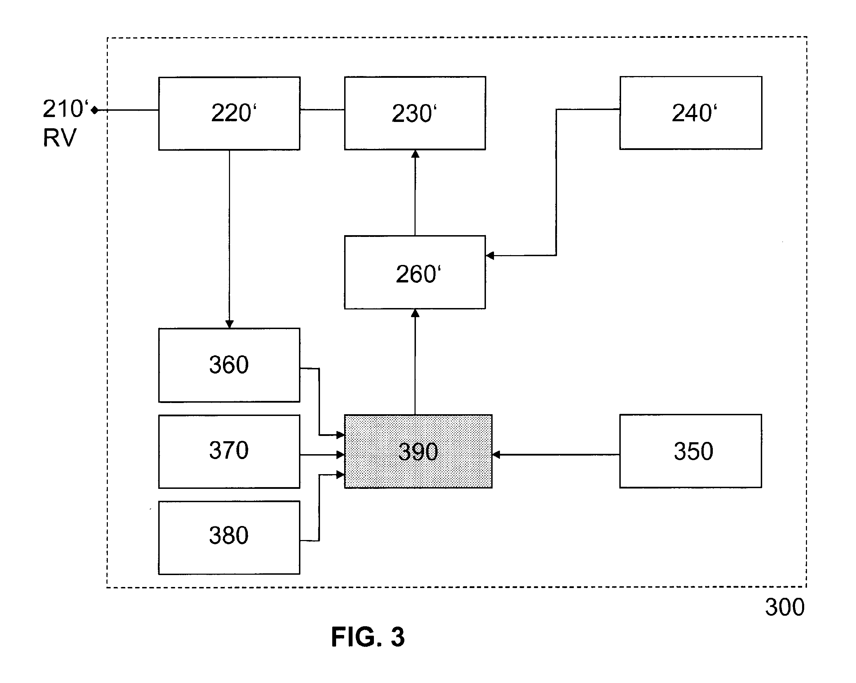

In der Figur 3 ist ein erweitertes Blockschaltbild dargestellt. Hier wird eine zusätzliche Kalibriereinheit (390) eingeführt. Diese dient der automatischen Bestimmung der liegenden Position des Patienten. Zu diesem Zweck ist diese Kalibriereinheit mit dem dreidimensionalen Lagesensor (350), einer Einheit zur Herzrhythmusanalyse (360), einer Einheit zur Atemfrequenzbestimmung (370) und der Implantatsuhr (380) verbunden.

Die Kalibriereinheit (390) speichert und aktualisiert immer dann Referenzdaten des Lagesensors (350), wenn die Parameter der Herzfrequenzanalyseeinheit (360: minimale Herzfrequenz, Herzfrequenzvariabilität), der Atemfrequenzanalyseeinheit (370) und die Implantatsuhrzeit (380: Vergleich mit Parametern Tag/Nachtumschaltung) einen Nachtschlag nahe legen.

Dieser aktuelle Referenzwert wird von der Kalibriereinheit (390) kontinuierlich mit dem aktuellen Wert des Lagesensors (350) verglichen und immer dann eine liegende Position der Steuereinheit (260') signalisiert, wenn der aktuelle und der Referenzwert übereinstimmen.

Die Erfindung ermöglicht es, die Spezifität einer automatischen MRT-Erkennung auf einfache Weise zu erhöhen. Dies erfolgt dabei mit einem sich automatisch kalibrierenden Lagesensor, so dass für den Anwender eine zusätzliche Kalibrierung dieses Sensors entfällt. Damit werden zudem fehlerhafte Sensorkalibrierungen ausgeschlossen.

## Patentansprüche

1. Implantierbares medizinisches Gerät (IMD) (200), mit
- einer Einheit zur Detektion von MRI-Störfeldern (240), wobei die Einheit über mindestens einen Sensor und/oder Indikator für MRI-typische elektromagnetische Störfelder oder MRI-typische Magnetfelder verfügt,
- mindestens einer Steuereinheit (260), die mit der Einheit zur Detektion von elektromagnetischen Störfeldern oder MRI-typischen Magnetfeldern (240) verbindbar ist, mindestens beinhaltend eine Diagnoseeinheit (360, 370) und/oder mindestens beinhaltend eine Therapieeinheit (220),
**gekennzeichnet dadurch, dass** die Einheit zur Detektion von MRI-Störfeldern (240) zusätzlich einen Lagesensor (250) aufweist und die Einheit zur Detektion von MRI-Störfeldern (240) nur dann eine Störung durch MRI-Störfelder erkennt, wenn neben zumindest einem Sensor und/oder zumindest einem Indikator für MRI-typische elektromagnetische Störfelder der Lagesensor (250) eine liegende Positur des Patienten anzeigt.

2. IMD nach Anspruch 1, **dadurch gekennzeichnet, dass** der Lagesensor (250) sich selber kalibriert, wobei eine liegende Positur entweder automatisch erkannt wird und/oder die Kalibrierung über ein externes Gerät, wie, aber nicht beschränkt auf, ein Patientengerät und/oder Programmiergerät, ausgelöst wird und/oder die liegende Positur über ein externes Gerät, wie, aber nicht beschränkt auf, ein Patientengerät und/oder Programmiergerät, bestätigt wird.

3. IMD nach einem der vorangegangenen Ansprüche, **dadurch gekennzeichnet, dass** die Einheit zur Detektion von MRI-Störfeldern (240) mindestens einen der folgenden Sensoren und/oder Indikatoren umfasst:
- GMR-Sensor,
- MagFET-Sensor,
- Hallsensor,
- elektrooptische Wandler als Indikator,
- die Überwachung von Batteriespannungen während Kondensatorlade-prozessen als Indikator,
- die Detektion von RF-Feldern als Indikator,
- die Detektion von magnetischen Grandientenfeldern als Indikator, und
- die Detektion von durch elektromagnetische Felder induzierten Strömen als Indikator.

4. IMD nach einem der vorangegangenen Ansprüche, **dadurch gekennzeichnet, dass** zumindest eine der folgenden Maßnahmen bei der Erkennung von MRI-Störfeldern durch die Einheit zur Detektion von MRI-Störfeldern (240) initiiert wird:
- das Wechseln in einen MRI-sicheren Zustand,
- ein verlängertes Verbleiben in einem MRI-sicheren oder gegenüber elektromagnetischen Störfeldern unempfindlichen Zustand,
- die Abgabe von elektromagnetischen Pulsen zur Signalisierung, dass ein IMD im elektromagnetischen Feld vorhanden ist, zur Signalisierung an ein MRI-Gerät, mit der Möglichkeit neben der Störung auch Informationen auf diese Weise zu übertragen und auf dem Bildschirm des MRI-Gerätes sichtbar zu machen, und
- die Abgabe einer Therapie und/oder Detektion von elektrischen Zuständen des Gewebes nur in Zeitfenstern erlaubt ist, in denen keine elektromagnetischen Störfelder erkannt werden und/oder eine Rekonstruktion einer Messung für die Bereiche durchgeführt wird, in denen die Detektion aufgrund von erkannten elektromagnetischen Störfeldern nicht erlaubt ist.

## Claims

1. An implantable medical device (IMD) (200), comprising
- a unit (240) for detecting MRI (magnetic resonance imaging) interference fields, wherein the unit has at least one sensor and/or indicator for electromagnetic interference fields typical for magnetic resonance imaging or magnetic fields typical for magnetic resonance imaging,
- at least one control unit (260), which can be connected to the unit (240) for detecting electromagnetic interference fields or magnetic fields typical for magnetic resonance imaging, at least containing a diagnostic unit (360, 370) and/or at least containing a treatment unit (220),
**characterised in that** the unit (240) for detecting MRI interference fields additionally has a position sensor (250) and the unit (240) for detecting MRI interference fields identifies interference by MRI interference fields only when, in addition to at least one sensor and/or at least one indicator for electromagnetic interference fields typical for magnetic resonance imaging, the position sensor (250) indicates a prone posture of the patient.

2. The IMD according to Claim 1, **characterised in that** the position sensor (250) is self-calibrating, wherein a prone posture is automatically identified and/or the calibration is triggered via an external device, such as, but not limited to, a patient device and/or programming device, and/or the prone posture is verified via an external device, such as, but not limited to, a patient device and/or programming device.

3. The IMD according to one of the preceding claims, **characterised in that** the unit (240) for detecting MRI interference fields comprises at least one of the following sensors and/or indicators:
- GMR sensor,
- MagFET sensor,
- Hall sensor,
- electro-optical converter as an indicator,
- the monitoring of battery voltages during capacitor charging processes as an indicator,
- the detection of RF fields as an indicator,
- the detection of magnetic gradient fields as an indicator, and
- the detection of currents induced by electromagnetic fields as an indicator.

4. The IMD according to one of the preceding claims, **characterised in that** at least one of the following measures is initiated with identification of MRI interference fields by the unit (240) for detecting MRI interference fields:
- change to an MRI-safe state,
- remain for a prolonged period of time in an MRI-safe state or a state that is insensitive to electromagnetic interference fields,
- emit electromagnetic pulses to signal (to an MRI device) that an IMD is present in the electromagnetic field, with the possibility of also transferring information in this way, in addition to the interference, and displaying this on the screen of the MRI device, and
- allow administration of treatment and/or detection of electrical states of the tissue only in time windows in which no electromagnetic interference fields are identified and/or carry out a reconstruction of a measurement for the regions in which the detection is not allowed on account of identified electromagnetic interference fields.

## Revendications

1. Dispositif médical implantable (IMD) (200), comprenant
- une unité (240) de détection de champs d'interférence d'IRM (imagerie par résonance magnétique), l'unité ayant au moins un capteur et/ou indicateur pour des champs d'interférence électromagnétiques typiques pour une imagerie par résonance magnétique ou des champs magnétiques typiques pour une imagerie par résonance magnétique
- au moins une unité de commande (260), qui peut être reliée à l'unité (240) de détection de champs d'interférence électromagnétiques ou de champs magnétiques typiques pour une imagerie par résonance magnétique, au moins contenant une unité de diagnostic (360, 370) et/ou au moins contenant une unité de traitement (220),
**caractérisé par le fait que** l'unité (240) de détection de champs d'interférence d'IRM a en outre un capteur de position (250) et l'unité (240) de détection de champs d'interférence d'IRM identifie une interférence par des champs d'interférence d'IRM seulement lorsque, en plus d'au moins un capteur et/ou au moins un indicateur pour des champs d'interférence électromagnétiques typiques pour une imagerie par résonance magnétique, le capteur de position (250) indique une position couchée du patient.

2. IMD selon la revendication 1, **caractérisé par le fait que** le capteur de position (250) est à auto-étalonnage, une position couchée étant automatiquement identifié et/ou l'étalonnage étant déclenché par l'intermédiaire d'un dispositif externe, tel que, mais sans y être limité, un dispositif de patient et/ou un dispositif de programmation, et/ou la position couchée étant vérifiée par l'intermédiaire d'un dispositif externe, tel que, mais sans y être limité, un dispositif de patient et/ou un dispositif de programmation.

3. IMD selon l'une des revendications précédentes, **caractérisé par le fait que** l'unité (240) de détection de champs d'interférence d'IRM comprend au moins l'un des capteurs et/ou indicateurs suivants :
- capteur GMR,
- capteur MagFET,
- capteur à effet Hall,
- convertisseur électro-optique comme indicateur,
- la surveillance de tensions de batterie pendant des procédés de charge de condensateur comme indicateur,
- la détection de champs de RF comme indicateur,
- la détection de champs de gradient magnétiques comme indicateur, et
- la détection de courants induits par des champs électromagnétiques comme indicateur.

4. IMD selon l'une des revendications précédentes, **caractérisé par le fait qu'**au moins l'une des mesures suivantes est amorcée par une identification de champs d'interférence d'IRM par l'unité (240) de détection de champs d'interférence d'IRM :
- changer vers un état de sécurité d'IRM,
- rester pendant une période de temps prolongée dans un état de sécurité d'IRM ou dans un état qui est insensible à des champs d'interférence électromagnétique,
- émettre des pulsations électromagnétiques pour signaler (à un dispositif IRM) qu'un IMD est présent dans le champ électromagnétique, avec la possibilité également de transférer des informations de cette manière, en plus de l'interférence, et d'afficher ceci sur l'écran du dispositif IRM, et
- permettre l'administration d'un traitement et/ou une détection d'états électriques du tissu seulement dans des fenêtres temporelles dans lesquelles il n'est pas identifié de champs d'interférence électromagnétiques et/ou effectuer une reconstruction d'une mesure pour les régions dans lesquelles la détection n'est pas permise en raison de champs d'interférence électromagnétiques identifiés.
